# DEMANDE DE BREVET EUROPEEN

(11) **EP 1 247 493 A1**
(43) Date de publication de la demande: **09.10.2002**
(21) Numéro de dépôt: 02356063.4
(22) Date de dépôt: 04.04.2002
(51) Int. Cl.: A61B 17/17

(54) **Guide de positionnement angulaire des os d'une articulation en vue de réaliser une arthrodése**

(30) Priorité: 04.04.2001 FR 0104592
(71) Demandeur: Newdeal S.A., 38200 Vienne (FR)
(72) Inventeur: Trnka, Hans-Jörg, 1170 Wien (AT)
(74) Mandataire: Martin, Didier Roland Valéry

(57) **Abrégé**

- Guide de positionnement angulaire des os d'une articulation en vue de réaliser une arthrodèse.
- L'invention concerne un guide de positionnement angulaire des os d'une articulation en vue de réaliser une arthrodèse comportant :
   - une embase (1) destinée à être positionnée sur l'articulation, et comportant un axe de réglage (2) dont la position est destinée à être réglée sensiblement à l'aplomb d'un premier repère géométrique fixe R de l'articulation,
   - un ensemble de réglage (10) monté à coulissement axial et à rotation sur l'axe de réglage (2), pour régler respectivement une hauteur de l'ensemble relativement au premier repère géométrique R et une première valeur angulaire a de l'ensemble autour dudit axe, ledit ensemble de réglage (10) étant formé d'un organe de réglage angulaire (11) qui est positionnable sur une pièce graduée (12) pour régler une seconde valeur angulaire β centrée sur le premier repère géométrique R.
- Guide de positionnement métatarso-phalangien.

## Description

La présente invention se rapporte au domaine technique général des appareils d'aide au positionnement angulaire des os d'une articulation en vue de réaliser une arthrodèse.

La présente invention concerne un guide de positionnement angulaire des os d'une articulation en vue de réaliser une arthrodèse.

Dans une application préférentielle, mais non exclusive, le guide de positionnement angulaire conforme à l'invention sera plus particulièrement destiné et conçu pour assurer le positionnement angulaire de l'articulation métatarso-phalangienne, étant entendu que des applications à d'autres articulations, notamment à celles de l'épaule, sont aussi envisageables.

Dans le cas d'arthrose survenant au niveau des articulations osseuses, et en particulier au niveau de l'articulation métatarso-phalangienne, il s'avère souvent nécessaire de pratiquer une arthrodèse afin de fusionner les deux os entre eux. De manière générale, les arthrodèses sont des opérations délicates, puisqu'en définitive elles bloquent une articulation dans une position définie et de manière irréversible. C'est ainsi que l'arthrodèse de l'articulation métatarso-phalangienne revêt un caractère hautement important, dans la mesure où cette articulation intervient de manière essentielle dans le cycle de la marche d'un être humain. On comprend alors qu'il s'avère essentiel que l'orientation entre les deux os, au moment de leur positionnement relatif avant la fusion, soit réalisée avec la meilleure précision possible pour éviter toute gêne ultérieure.

Il s'avère donc essentiel de fixer les axes relatifs des os pour respecter au mieux les flexions et axes du patient, et ce en fonction du sexe du patient et de sa morphologie.

Jusqu'à présent, les arthrodèses métatarso-phalangiennes sont réalisées de manière relativement empirique, la détermination et la fixation des valeurs angulaires entre le métatarse et les phalanges étant réalisées de manière relativement grossière, qu'il s'agisse de la détermination de l'angle en varus-valgus ou de l'angle en dorsiflexion de l'articulation.

En pratique, on constate que si les angles choisis par le chirurgien sont mal respectés en cours d'opération, et aboutissent en conséquence à une fixation incorrecte des axes de fusion entre les os, on aboutit soit à une gêne du patient lors de la marche, qu'il compense alors par un positionnement de correction susceptible de causer lui-même des troubles secondaires, soit à une gêne au chaussage ou au déchaussage, soit encore à des frottements ou irritations à l'origine de plaies diverses.

Dans tous les cas, le non respect des angles de fusion, soigneusement déterminés au préalable par le chirurgien, entraîne des conséquences néfastes pour le patient subissant une arthrodèse, qu'il convient précisément d'éviter.

L'objet de l'invention vise précisément à porter remède aux différents inconvénients énumérés précédemment, et à proposer un appareil ou guide de positionnement angulaire des os d'une articulation en vue de réaliser une arthrodèse.

Un autre objet de l'invention vise à proposer un guide de positionnement particulièrement adapté pour assurer, de manière simple et fiable, le positionnement angulaire de l'articulation métatarso-phalangienne.

Un autre objet de l'invention vise à proposer un guide de positionnement dont le réglage initial et la mise en place sont particulièrement aisés.

Un autre objet de l'invention vise à proposer un guide de positionnement permettant le réglage de différents axes de manière sûre et rapide.

Les objets assignés à l'invention sont atteints à l'aide d'un guide de positionnement angulaire des os d'une articulation, en vue de réaliser une arthrodèse comportant :
- une embase destinée à être positionnée sur l'articulation, et comportant un axe de réglage dont la position est destinée à être réglée sensiblement à l'aplomb d'un premier repère géométrique fixe de l'articulation,
- un ensemble de réglage monté à coulissement axial et à rotation sur l'axe de réglage, pour régler respectivement une hauteur de l'ensemble relativement au premier repère géométrique et une première valeur angulaire α de l'ensemble autour dudit axe, ledit ensemble de réglage étant formé d'un organe de réglage angulaire qui est positionnable sur une pièce graduée pour régler une seconde valeur angulaire β centrée sur le premier repère géométrique, mais située dans un plan différent de la première valeur angulaire α.

D'autres avantages et objets de l'invention apparaîtront plus en détails à la lecture de la description qui suit, et à l'aide des dessins annexés fournis à titre purement explicatif et non limitatif, dans lesquels :
- La figure 1 illustre, selon une vue en perspective, une vue générale du guide de positionnement angulaire des os d'une articulation conforme à l'invention.
- La figure 2 illustre, selon une vue de dessus, le réglage de l'angle en varus-valgus dans le cas d'une arthrodèse de l'articulation métatarso-phalangienne.
- La figure 3 illustre, selon une vue latérale, le réglage de l'angle en dorsiflexion d'une articulation métatarso-phalangienne.
- La figure 4 illustre, selon une vue latérale, une vue de détail d'un élément de centrage utilisé avec un guide de positionnement conforme à l'invention.

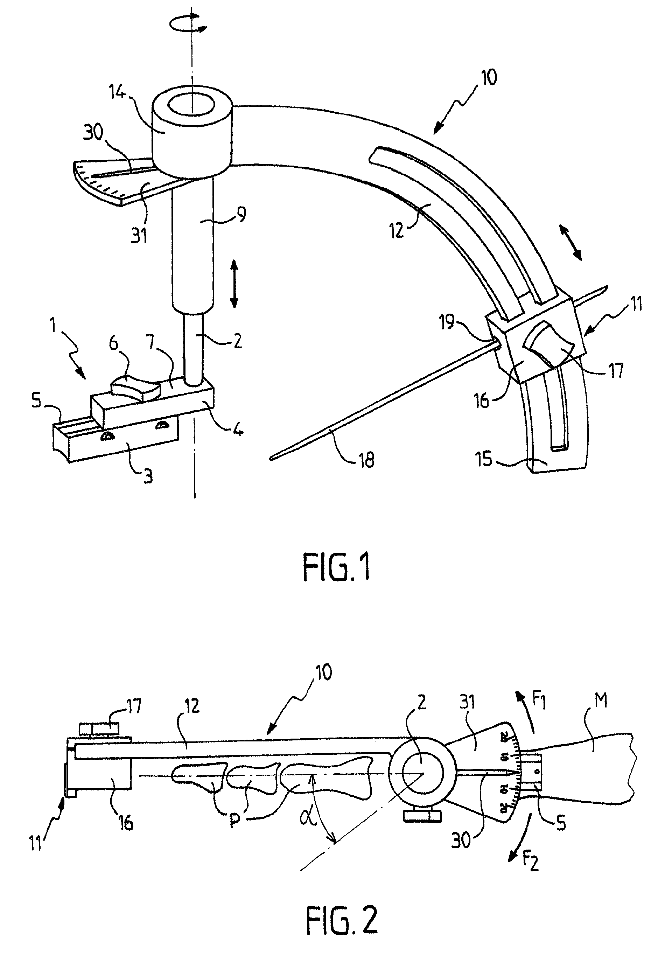

L'ancillaire ou le guide de positionnement angulaire des os d'une articulation, en vue de réaliser une arthrodèse, correspondant à l'invention et illustré aux figures 1 à 3, est destiné à assurer plus particulièrement le positionnement angulaire de l'articulation métatarso-phalangienne d'un être humain.

Néanmoins, on comprend que des applications au positionnement angulaire des os d'un autre type d'articulation sont envisageables, et c'est la raison pour laquelle le nouvel ancillaire développé ne sera en aucune façon limité à une arthrodèse de l'articulation métatarso-phalangienne, bien que pour les besoins de la description de la présente demande de brevet, l'application à ce type d'articulation spécifique sera décrite.

Ainsi, tel qu'illustré aux figures, le guide de positionnement angulaire comprend une embase 1 destinée à être positionnée sur l'articulation et, dans le cas, présent sur le métatarse M de l'articulation métatarso-phalangienne. L'embase 1 comporte un axe de réglage 2 s'étendant sensiblement verticalement, lorsque l'embase 1 est mise en place sur le métatarse M, et dont la position est destinée à être réglée sensiblement à l'aplomb d'un premier repère géométrique fixe R de l'articulation.

Tel qu'illustré, et dans son application préférentielle à une articulation métatarso-phalangienne, le premier repère géométrique fixe R sera le centre de rotation de la tête articulaire du métatarse M.

Dans la variante préférentielle illustrée aux figures 1 à 3, l'embase sera avantageusement formée par une platine principale 3 pourvue de moyens de fixation (non représentés aux figures), et par exemple d'orifices traversants pour permettre l'introduction de vis permettant la fixation à demeure sur un os de l'articulation. Selon cette variante préférentielle, l'embase 1 est également formée par une platine secondaire 4 montée mobile sur la platine principale 3, ladite platine secondaire 4 supportant l'axe de réglage 2, de manière à pouvoir régler la position dudit axe relativement au centre de rotation R du métatarse M.

La platine secondaire 4 peut être montée mobile sur la platine principale 3 par tous moyens techniques bien connus de l'homme du métier, et par exemple par l'intermédiaire d'un montage à glissière 5 associé à un organe de blocage 6, tel qu'un bouton rotatif.

De cette façon, le chirurgien peut, après avoir au préalable fixé la platine principale 3 sur le métatarse M, amener facilement et rapidement, par simple coulissement de la platine secondaire 4, l'axe de réglage 2 à l'aplomb du repère géométrique R. Une fois ce déplacement réalisé, la platine secondaire 4 est bloquée en position par l'intermédiaire du bouton de réglage 6.

Bien évidemment, à titre de variante, il est tout à fait envisageable de réaliser le dispositif conforme à l'invention avec une embase 1 formée d'une platine unique, l'opération de positionnement de l'axe de réglage 2 étant alors plus délicate à réaliser par le chirurgien, puisqu'elle est alors associée à un positionnement et à une fixation simultanés précis de ladite embase.

Dans les exemples illustrés, la platine principale 3 et la platine secondaire 4 sont réalisées sous la forme de pièces sensiblement parallélépipédiques, définissant des faces respectives de glissement sensiblement planes, la platine secondaire 4 comportant une face supérieure 7 à partir de laquelle s'étend, de manière sensiblement perpendiculaire au plan d'extension de ladite face 7, l'axe de réglage 2.

Le guide de positionnement angulaire conforme à l'invention comporte également un ensemble de réglage 10 qui est monté à coulissement axial et à rotation directement sur l'axe de réglage 2, pour régler respectivement une hauteur dudit ensemble 10 relativement au premier repère géométrique fixe R, et une première valeur angulaire α dudit ensemble 10 autour dudit axe 2.

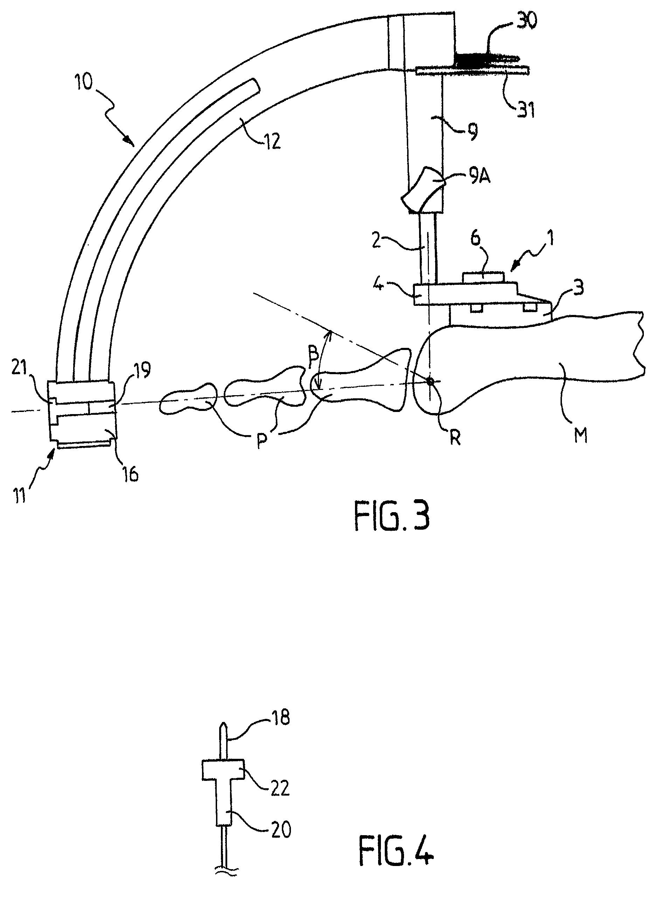

Avantageusement, l'ensemble de réglage 10 comprend un manchon tubulaire creux 9 pourvu d'une vis de blocage 9A, ledit manchon étant enfilé dans ou sur (figure 3) l'axe 2. Ce dernier peut être pourvu d'un méplat pour favoriser le blocage en rotation.

Dans l'application à une articulation métatarso-phalangienne, la rotation de l'ensemble de réglage 10 servira à régler l'angle de varus-valgus qui correspondra donc à la valeur angulaire α. Ce réglage peut s'opérer par l'intermédiaire d'un repère 30 et d'une plaque graduée 31, l'un étant lié à l'axe 2 et l'autre au manchon 9 ou à l'ensemble de réglage 10 par exemple.

Selon cette disposition, l'ensemble de réglage 10 comporte un organe de réglage angulaire 11, qui est positionnable sur une pièce graduée 12 pour régler une seconde valeur angulaire β centrée sur le premier repère géométrique fixe R, mais située dans un plan différent de la première valeur angulaire α.

Dans le cas d'une articulation métatarso-phalangienne, le guide selon l'invention sera réalisé de telle façon que l'axe de réglage 2 est perpendiculaire au plan d'extension de l'embase 4, et l'organe de réglage 11 ainsi que la pièce graduée 12 sont agencés pour régler la seconde valeur angulaire β selon un plan qui est perpendiculaire à celui dans lequel la première valeur angulaire α est réglée.

Ainsi, le guide selon l'invention permet de définir un centre de référence R par rapport auquel on règle un système de référence géométrique complet, à savoir par l'intermédiaire de l'axe de réglage 2, un plan de référence puis dans ce plan, une première valeur angulaire α à rotation autour de l'axe de réglage 2, puis une seconde valeur angulaire β par déplacement et positionnement de l'organe de réglage 11. On obtient ainsi un repère géométrique, de préférence orthonormé, complet.

Tel qu'illustré aux figures, l'ensemble de réglage 10 est monté sur l'axe de réglage 2 par l'intermédiaire de la pièce graduée 12, qui est supportée à rotation sur l'axe de réglage 2 par l'une de ses extrémités 14 prolongée par le manchon 9.

Avantageusement, la pièce graduée 12 est formée par une règle en forme de quart de cercle s'étendant dans un plan perpendiculaire au plan d'extension de l'embase 1, l'extrémité libre 15 de la règle étant située à un niveau inférieur à l'extrémité de montage 14 sur l'axe de réglage 2. Ceci permet donc de définir une possibilité de réglage angulaire de l'angle β d'une amplitude de 45 °.

Tel qu'illustré aux figures, l'organe de réglage angulaire 11 comporte un curseur 16 qui est monté déplaçable sur la pièce graduée 12 pourvue d'une fente ménagée dans l'épaisseur de ladite pièce. Le curseur 16 est associé à un organe de blocage / déblocage 17 monté sur ledit curseur 16, adapté pour être manipulé par le chirurgien, et servant à déplacer puis à fixer la position angulaire du curseur 16.

Le curseur 16 est adapté pour supporter une broche chirurgicale 18 qui est apte à être enfilée axialement à travers les différents os formant les phalanges de l'articulation (figure 2), en vue de réaliser l'arthrodèse.

En effet, pour régler la valeur angulaire β correspondant à l'angle de dorsiflexion de l'articulation métatarso-phalangienne, le chirurgien doit tout d'abord aligner axialement l'ensemble des os de l'articulation à l'aide de la broche chirurgicale 18, qui a, au préalable, été enfilée à travers les os. Par la suite, le chirurgien doit venir fixer, dans l'espace, l'angle de dorsiflexion recherché pour réaliser l'arthrodèse en elle-même, ce qui implique de disposer d'un support fixe de la broche chirurgicale 18 à la valeur de l'angle β sélectionnée.

Pour réaliser cette fonction de support, le curseur 16 est pourvu sur l'une de ses faces d'une gorge 19, par exemple de forme hémicirculaire, de dimensions suffisantes pour pouvoir supporter de manière stable une portion de longueur de la broche chirurgicale 18, tout en permettant l'introduction latérale de la broche 18 qui a été au préalable enfilée à travers les phalanges.

Avantageusement, pour faciliter l'introduction et améliorer le support stable de la broche chirurgicale 18 dans la gorge 19, il est prévu un centreur 20 (figures 3 et 4) destiné à être enfilé et bloqué en position sur l'extrémité libre de la broche 18 après son introduction à travers les phalanges. La gorge 19 présente à cet effet une découpe spécifique de forme et de dimensions appropriées et correspondant à celle du centreur 20, de manière à ce que ce dernier puisse être introduit dans la gorge pour être supporté de manière stable dans ledit logement 21.

Tel qu'illustré aux figures 3 et 4, le centreur se présente sous la forme d'une petite pièce cylindrique avec un canal longitudinal d'insertion, ladite pièce cylindrique étant surmontée par une tête de dimensions supérieures formant une collerette 22. Le logement 21 (figure 3) est de forme identique et correspondante au centreur 20 ainsi défini.

Le principe de fonctionnement du guide de positionnement métatarsophalangien conforme à l'invention est le suivant.

Après avoir fait une incision au niveau de l'articulation métatarso-phalangienne, le praticien commence par mettre en place une broche chirurgicale 18 en l'enfilant à travers l'ensemble des phalanges P, tel qu'illustré partiellement à la figure 3. De manière classique, la mise en place de la broche chirurgicale s'effectue par exemple au moteur, à partir de la face articulaire vers l'extérieur, de telle façon que la pointe de la broche chirurgicale 18 soit affleurante à la surface articulaire. Le chirurgien peut alors dès maintenant, ou ultérieurement, introduire axialement et bloquer en position le centreur 20 à l'extrémité libre de la broche chirurgicale 18.

Le chirurgien assure ensuite la mise en position du guide conforme à l'invention en positionnant l'embase 1 sur la face supérieure du métatarse M.

A cette fin, il pose et fixe, par exemple par l'intermédiaire de broches, de vis ou de pinces de fixation, la platine principale 3 sensiblement vers l'extrémité du métatarse M. Par la suite, le chirurgien débloque le bouton 6 pour permettre la translation de la platine secondaire 4 et régler la position relative de l'axe de réglage 2 vis à vis du centre de rotation du métatarse M, de façon à ce que l'axe de réglage 2 soit à l'aplomb de ce dernier. Le praticien assure ensuite le blocage de l'axe de réglage 2 par manipulation du bouton 6. Ce premier réglage correspond ainsi à une translation parallèle à l'axe principal du métatarse.

Par la suite, le praticien assure la mise en place de l'ensemble de réglage 10 qui est alors enfilé sur l'axe de réglage 2, dont il convient désormais de régler la position verticale le long de l'axe de réglage 2. La translation verticale s'effectue jusqu'à ce que le praticien positionne la position de référence 0 du curseur 16, correspondant sensiblement à l'extrémité 15 de la pièce graduée 12, à la même hauteur que le centre de rotation R de la tête articulaire du métatarse M (figure 3). Une fois cette position de référence effectuée, l'ensemble de réglage 10 est bloqué en position par manipulation du bouton de blocage associé à l'axe 2.

En ayant effectué ces deux réglages en translation, le chirurgien est alors assuré que toutes les rotations angulaires qui seront faites ultérieurement auront un centre confondu avec le centre de rotation du métatarse R, qui forme donc le premier repère géométrique fixe de l'articulation.

Par la suite, selon la morphologie du patient et selon le fait qu'il s'agisse d'un homme ou d'une femme, le praticien va régler les deux positions angulaires nécessaires α et β pour pratiquer une arthrodèse de l'articulation métatarso-phalangienne, à savoir tout d'abord l'angle en varus-valgus (angle interne), puis l'angle de dorsiflexion. En général, l'angle de varus-valgus est réglé avec une valeur angulaire α de l'ordre de 15 à 25 °, alors que l'angle de dorsiflexion est réglé avec une valeur angulaire β de l'ordre de 20 à 30 °.

L'angle en varus-valgus est alors réglé en faisant pivoter l'ensemble de réglage 10 autour de l'axe de réglage 2 selon l'une ou l'autre des flèches F1, F2 montrées à la figure 2, selon qu'il s'agit d'une opération effectuée avec un patient côté droit ou côté gauche. L'angle en varus-valgus recherché est obtenu en repérant la position du repère 30 sur la plaque graduée 31 (figure 2), sur laquelle des valeurs angulaires ont été reportées. Après l'atteinte de la valeur angulaire α de l'angle en varus-valgus souhaitée, le praticien bloque à nouveau l'ensemble de réglage 10 sur l'axe de réglage 2.

La valeur angulaire de l'angle β correspondant à l'angle de dorsiflexion est ensuite réglée à l'aide du curseur 16, qui est amené en position sur la pièce graduée 12 en regard de la valeur angulaire souhaitée, le curseur 16 étant ensuite à nouveau bloqué en position à la valeur angulaire β souhaitée.

Le praticien peut alors mettre en position la broche 18 dans la gorge 19 en insérant le centreur 20 dans le logement 21, ce qui fixe dans l'espace la position relative des os de l'articulation métatarso-phalangienne dans laquelle l'arthrodèse devra être effectuée.

De cette façon, le positionnement des os de l'articulation est rigoureusement fixé, et il ne reste plus au praticien qu'à introduire la broche chirurgicale 18 dans la tête du métatarse pour fusionner ensuite les deux parties osseuses. Par la suite, le chirurgien peut retirer le guide conforme à l'invention en laissant simplement la broche chirurgicale 18 en place.

## Revendications

1. Guide de positionnement angulaire des os d'une articulation telle que notamment l'articulation métatarso-phalangienne en vue de réaliser une arthrodèse comportant
- une embase (1) destinée à être positionnée sur l'articulation, et comportant un axe de réglage (2) dont la position est destinée à être réglée sensiblement à l'aplomb d'un premier repère géométrique fixe R de l'articulation,
- un ensemble de réglage (10) monté à coulissement axial et à rotation sur l'axe de réglage (2), pour régler respectivement une hauteur de l'ensemble relativement au premier repère géométrique R et une première valeur angulaire α de l'ensemble autour dudit axe, ledit ensemble de réglage (10) étant formé d'un organe de réglage angulaire (11) qui est positionnable sur une pièce graduée (12) pour régler une seconde valeur angulaire β centrée sur le premier repère géométrique R, mais située dans un plan différent de la première valeur angulaire α.

2. Guide selon la revendication 1 **caractérisé en ce que** l'axe de réglage (2) est perpendiculaire au plan d'extension de l'embase (1), et l'organe de réglage angulaire (11) et la pièce graduée (12) sont agencés pour régler la seconde valeur angulaire β selon un plan perpendiculaire à celui dans lequel la première valeur angulaire α est réglée.

3. Guide selon la revendication 1 ou 2 **caractérisé en ce que** l'embase (1) est formée par une platine principale (3) pourvue de moyens de fixation, pour être fixée à demeure sur un os de l'articulation, et par une platine secondaire (4), montée mobile sur la platine principale (3) et supportant l'axe de réglage (2) de manière à pouvoir régler la position dudit axe.

4. Guide selon l'une des revendications 1 à 3 **caractérisé en ce que** l'ensemble de réglage (10) est monté sur l'axe de réglage (2) par l'intermédiaire de la pièce graduée (12) supportée à rotation sur l'axe (2) par l'une de ses extrémités (14).

5. Guide selon la revendication 4 **caractérisé en ce que** la pièce graduée (12) est formée par une règle en forme de quart de cercle s'étendant dans un plan perpendiculaire au plan d'extension de l'embase (1), l'extrémité libre de la règle (15) étant située à un niveau inférieur à l'extrémité (14) de montage.

6. Guide selon l'une des revendications 1 à 5 **caractérisé en ce que** l'organe de réglage angulaire (11) est un curseur déplaçable (16).

7. Guide selon la revendication 6 **caractérisé en ce que** le curseur déplaçable (16) est adapté pour supporter une broche chirurgicale (18).

8. Guide selon la revendication 7 **caractérisé en ce que** le curseur (16) est pourvu d'une gorge (19) permettant l'introduction latérale et le support de la broche (18).

9. Guide selon la revendication 8 **caractérisé en ce qu'**il comporte un centreur (20) destiné à être enfilé et bloqué en position sur une broche chirurgicale (18), et à être introduit dans la gorge (19) pour être supporté dans un logement (21) ménagé dans le curseur (16).
